# EUROPEAN PATENT APPLICATION

(11) **EP 1 484 046 A1**
(43) Date of publication of application: **08.12.2004**
(21) Application number: 03012867.2
(22) Date of filing: 06.06.2003
(51) Int. Cl.: A61K 6/083

(54) **Homogeneous dental composite material and method of preparation**

(71) Applicant: Degussa AG, 40474 Düsseldorf (DE)
(72) Inventor: Scholz, Mario, 63584 Gründau (DE); El Moussaoui, Aziz, 63457 Hanau (DE); Mörters, Martin, Dr., 79618 Rheinfelden (DE)

(57) **Abstract**

Homogeneous dental composite material comprising fumed silica powder and a resin, the resin comprising a polymerizable monomer, the fumed silica powder has a BET surface area 30 to 90 m²/g, a DBP number of 80 or less, an average aggregate area of less than 25,000 nm², and in which at least 70% of the aggregates have a diameter of less than 1300 nm and is present from at least 10 wt.%, based on the total dental composite material.

## Description

### BACKGROUND OF THE INVENTION

This invention relates to a homogeneous composite material for use in dental restorations and for layering over microhybrids.

Dental composite materials are generally produced by mixing finely divided silica with a polymerizable monomer, usually an acrylate or methacrylate-based resin, heat polymerizing the mixture in bulk, and pulverizing the mixture down to the desired agglomerate size to give a filler material comprised of splintered polymerized particles. This filler material is then mixed with a polymerizable monomer, again typically an acrylate or methacrylate-based resin, and an additional filler material, such as colloidal silica. Thus, there are typically two polymerization steps, with the first being referred to as prepolymerization.

For example, U.S. Pat. No. 4,781,940 describes a process for producing a dental composite material using a prepolymerization step. A slurry of silica in a polymerizable monomer/solvent solution is prepared, followed by evaporating the solvent by heating at atmospheric pressure. The monomer coated silica particles are individualized by sieving, then polymerized by heating, and again sieved. No pulverization step is required. This filler material is then mixed with an additional filler and a resin, which may have the same monomers as those used in the monomer/solvent solution.

The composite material resulting from processes such as that of U.S. Pat. No. 4,781,940 is heterogeneous as a result of the prepolymerization step. Heterogeneous dental fillers are hydrolytically unstable and suffer from catastrophic marginal failures when used in dental restorations, in part, as a result of separation along the prepolymerized particle-resin matrix interface caused by percolation of aqueous fluids. Thus, it is desirable to develop a dental composite material that does not involve prepolymerization of the silica particles.

U.S. Pat. No. 4,389,497 is directed to a filler material which may or may not include a prepolymerization step, and therefore, may be heterogeneous or homogeneous. The inorganic filler material used is in the form of agglomerates of silicic acid, which can be produced with or without a binding agent. For example, the agglomerates could be formed by premixing silicic acid with a water glass solution, a boric acid solution, or an alcoholic aluminum alcoholate solution. The agglomerated material is then adjusted to the desired size by milling and screening. A further manner of production involves premixing the silicic acid with organo-silicon compounds preferably containing a polymerizable residue and a polymerization catalyst if necessary, followed by heat polymerization. The organic constituents are then burned and the mixture is brought up to more than 600 °C. Agglomerate reduction to the desired size is then achieved by milling and screening, subsequent to the heating step.

In addition to the homogeneous composites disclosed in U.S. Pat. No. 4,389,497, there are other homogeneous composites that have been offered commercially. These homogeneous composites have proved unsuccessful in that they are difficult to prepare reproducibly and the theological properties are difficult to control. This is due to difficulty in controlling the agglomeration of silica particles. Thus, it is desirable to develop a process in which the agglomeration can be controlled to prepare reproducibly a homogeneous composite material.

The US patent 6,020,395 describes a method using a homogeneous dental composite material, which contains as a filler a fumed silica, for example Aerosil OX50. The silica is then coated with an organosilane, milled and oxidized at elevated temperatures to form a fused silica, which has a tendency to associate with itself to form siloxane units. In a following step the fused silica is contacted with an organosilane to form a silane treated fused silica, followed by drying and sieving. In a final step the silane-treated fused silica is combined with a resin consisting of a polymerizable momomer and an additional fumed silica to form the composite dental material.

EP-A-1290997 describes a silica dental composite material comprising functionalized and/or functionalized, structurally modified silica, the functional groups fixed on the surface being 3-methacryloxypropylsilyl and/or glycidyloxypropylsilyl groups and the silica having a BET surface area of from 20 to 380 m²/g, a tamped density of from 50-600 g/l, a pH value of from 3 to 10, a carbon content of from 0.1 to 15 wt.% and a DBP number of less than 200. In a following step a homogeneous resin mixture consisting of a polymerizable organic acrylic monomer is prepared and mixed the functionalized silica. In contrast to US 6,020,395 no fused silica is formed.

Although the prior art shows numerous documents using silicas as part of dental composite materials, there is still a need to improve the mechanical properties of these materials, which critically depend on the filling degree of the silica in the composite material.

It is therefore an object of the present invention to provide a homogeneous dental composite material having a high content of silica.

The present invention provides a homogeneous dental composite material comprising polymerizable monomer mixed with a high content of silica particles for use in dental restorations, and a method for making the same.

Subject of the invention is a homogeneous dental composite material comprising fumed silica powder and a resin, the resin comprising a polymerizable monomer, characterized in that the fumed silica powder
- has a BET surface area 30 to 90 m²/g, a DBP number of 80 or less, an average aggregate area of less than 25,000 nm², and in which at least 70% of the aggregates have a diameter of less than 1300 nm and
- is present from at least 10 wt.%, based on the total dental composite material.

A fumed silica powder according to the invention is described in the yet unpublished German patent application DE 10258857.0, December 17, 2002.

In a preferred embodiment, the BET surface area of the fumed silica powder used for the inventive homogeneous dental composite material may be between 35 and 75 m²/g. Particularly preferably the values may be between 40 and 60 m²/g. The BET surface area is determined in accordance with DIN 66131.

The DBP index of the fumed silica powder used for the inventive homogeneous dental composite material may, in a preferred embodiment, be between 60 and 80. During DBP absorption, the take-up of force, or the torque (in Nm), of the rotating blades in the DBP measuring equipment is measured while defined amounts of DBP are added, comparable to a titration. A sharply defined maximum with a subsequent drop at a specific added amount of DBP is then produced for the powder according to the invention.

In another preferred embodiment, the fumed silica powder in the inventive homogeneous dental composite material may have a mean aggregate area of at most 20000 nm². Particularly preferably, the mean aggregate area may be between 15000 and 20000 nm². The aggregate area can be determined, for example, by image analysis of TEM images. An aggregate in the context of the invention is understood to consist of primary particles of similar structure and size which have intergrown with each other, the surface area of which is less than the sum of the individual isolated primary particles. Primary particles, in the context of the invention, are understood to be particles which are initially formed in the reaction and which can grow together to form aggregates as the reaction proceeds further.

In another preferred embodiment, the fumed silica powder in the inventive homogeneous dental composite material may have a mean aggregate circumference of less than 1000 nm. Particularly preferably, the mean aggregate circumference may be between 600 and 1000 nm. The aggregate circumference can also be determined by image analysis of TEM images.

An embodiment may be preferred in which at least 80%, particularly preferably at least 90%, of the aggregates have a circumference of less than 1300 nm.

The fumed silica powder can be produced by a process in which at least one silicon compound in the vapour form, a free-oxygen-containing gas and a combustible gas are mixed in a burner of known construction. This gas mixture is ignited at the mouth of the burner and is burnt in the flame tube of the burner, the solid obtained is separated from the gas mixture and optionally purified. The oxygen content of the free-oxygen-containing gas has to be adjusted so that the lambda value is greater than or equal to 1, the gamma-value is between 1.2 and 1.8, the throughput is between 0.1 and 0.3 kg SiO₂/m³ of core gas mixture, and the mean normalised rate of flow of gas in the flame tube at the level of the mouth of the burner is at least 5 m/s. Here, lambda describes the ratio of oxygen supplied in the core to the stoichiometrically required amount of oxygen. Gamma describes the ratio of hydrogen supplied in the core to the stoichiometrically required amount of hydrogen. The normalised rate of flow of gas refers to the rate of flow at 273 K and 1 atm. A burner of known construction is understood to be a burner consisting of concentric tubes. The core gases are passed through the internal tube, the core. At the end of the tube, the mouth of the burner, the gases are ignited. The internal tube is surrounded by at least one other tube, the sleeve. The reaction chamber, called the flame tube, starts at the level of the mouth of the burner. This is generally a conical tube, cooled with water, which may optionally be supplied with other gases such as hydrogen or air. The core gases are understood to be the gases and vapours supplied to the burner, that is the free-oxygen-containing gas, generally air or air enriched with oxygen, the combustible gas, generally hydrogen, methane or natural gas, and the silicon compound or compounds in vapour form. The type of silicon compound used in the process according to the invention is not further restricted. Silicon tetrachloride and/or at least one organochlorosilicon compound may preferably be used.

In a preferred embodiment the inventive homogeneous dental composite material contains 15 to 50 wt.% of the fumed silica powder, based on the total composite material.

In a another preferred embodiment the inventive homogeneous dental composite material contains 20 to 35 wt.% of the fumed silica powder, based on the total composite material.

Furthermore the homogeneous dental composite material according to the invention may contain additional fillers like silica, functionalized silica, functionalized, structurally modified silica and/or treated glass.

The polymerizable monomer may be an acrylate or a mixture of acrylates. These monomers that may be used in the initiator and/or accelerator pastes of the present invention are well known in the art. See, e.g. U.S. Pat. No. 4,383,826; U.S. Pat. No. 4,333,348; and U.S. Pat. No. 3,066,112, these patents being incorporated herein by reference. Thus, some non-limiting examples of the polymerizable acrylate monomers which may be used include acrylic acid, methacrylic acid; typical alkyl methacrylates (e.g. methyl methacrylate, butyl methacrylate); cycloaliphatic methacrylates (e.g. cyclohexyl methacrylate; iso-bornyl methacrylate); aryl methacrylates (phenyl methacrylate; benzyl methacrylate; bisphenol dimethacrylates, etc.); functional methacrylates (such as 2-hydroxyethyl methacrylate; 4-methacryloxyethoxybenzaldehyde; 4-methacryloxyethoxybenzoic acid); ethylene glycol dimethacrylate; diethylene glycol dimethacrylate; triethylene glycol dimethacrylate; tetraethylene glycol dimethacrylate; polyethylene glycol and polypropylene glycol dimethacrylates; ethoxylated and propoxylated bisphenol dimethacrylates; neopentyl glycol dimethacrylate; trimethylolpropane trimethacrylate; 1,6-hexanediol-, 1,10-decanediol and 1,4-cyclohexanediol dimethacrylates; pentaerythritol tetramethacrylate; 1,10-decamethylene dimethacrylate; 1H, 1H-pentadecafluoroctyl methacrylate; 2,2-bis[p-(2'-hydroxy-3-methacryloxypropoxy)phenyl]propane, i.e. bis-GMA; and various nonhydroxylated homologs of bis-GMA e.g. 2,2-bis[p-(2'-methacryloxyethoxy)phenyl]propane; various diurethane dimethacrylates (e.g. the diadduct of 2-hydroxyethyl methacrylate and trimethylhexamethylene diisocyanate), oligomeric urethanes with multifunctional methacrylate groups (e.g. urethane derivatives of bis-GMA and aliphatic and cycloaliphatic diisocyanates), and other prepolymer types of monomers (e.g. siloxane multifunctional methacrylates; polyfluorinated oligomeric multifunctional methacrylates, etc.) and the like. Mixtures of the above and other acrylate monomers or other copolymerizable monomers (such as any vinyl monomer capable of free radical polymerization, e.g. styrene, alpha-methylstyrene, vinyl biphenyl, vinyl acetate, pentafluorostyrene, and similar olefinic monomers) also may be used in either or both of the paste components. However, bulky multifunctional acrylic monomers are preferred since they have relatively low exotherms and shrinkages on polymerization, and because of their crosslinking nature, yield materials with low water sorption, high strength and excellent dimensional stability. In addition such monomers are desirable because of their low volatility and minimum potential for tissue irritation.

A preferred homogeneous resin may be produced by mixing the following components (in parts by weight):
- 60-90 parts Diurethane Dimethacrylate (Rohamere 6661-0, Huls America, Inc. Piscataway, N.J.),
- 10-40 parts Triethyleneglycoldimethacrylate,
- 0.4-1.0 parts 2-Hydroxy-4-methoxybenzophenone,
- 0.07-0.4 parts Camphorquinone,
- 0.1-1.0 parts p-octyl dimethylamino-benzoate.

This cured resin system is tough and highly cross-linked. The goal in selecting a resin system is to provide a resin with a refractive index similar to that of silica, such that they are a relatively good match from a visual and aesthetic standpoint.

Another object of the invention is a method to produce the homogeneous dental composite material comprising the steps:
- combining a resin comprising a polymerizable monomer and the fumed silica powder
- optionally adding an additional filler and
- optionally curing the mixture.

The additional filler and the fumed silica may be dispersed into the resin until the mixture is uniform, as a rule 3 to 10 hours. For dispersing a planetary mixer may be used. Preferably, the mixture is then deaerated for at least 10 minutes in a vacuum.

The homogeneous dental composite material of the present invention exhibits due to its high content of fumed silica powder better physical properties than the currently marketed dental composite materials. For example, substantially higher flexural strength and toughness are obtained with the present invention. This assures higher fracture resistance than a typical dental composite material. This high strength and fracture resistance help reduce gouging during finishing.

A further advantage of the present invention is that the composite material is highly polishable and can be brought rapidly to a high luster, which is essential for materials used in exterior tooth applications. Furthermore, this high luster is retained after tooth brushing and chewing.

### Examples

The physico-chemical data of the fumed silicas and the dental composite materials are determined as described in the German patent application DE 10258857.0, December 17, 2002 and in EP-A-1290997.

### Fumed Silica Powders

**Example A:** 400 kg/h SiCl₄ are vaporised at about 90°C and transferred to the central tube of a burner of known construction. 195 Nm³/h of hydrogen and 303 Nm³/h of air with an oxygen content of 30 vol% are also introduced into this tube. This gas mixture is ignited and burnt in the flame tube of the water-cooled burner. The mean normalised rate of flow of gas in the flame tube at the level of the mouth of the burner is 10 m/s. After cooling the reaction gases, the fumed silica powder is separated from the hydrochloric acid-containing gases using a filter and/or a cyclone. The fumed silica powder A is treated with water vapour and air in a deacidification unit.

**Example B:** Powder B is prepared in the same way as described in example A. The experimental conditions are given in Table 1.

**Example C:** 500 kg/h SiCl₄ are vaporised at about 90°C and transferred to the central tube of a burner of known construction. 145 Nm³/h of hydrogen and 207 Nm³/h of air with an oxygen content of 35 vol% are also introduced into this tube. This gas mixture is ignited and burnt in the flame tube of the water-cooled burner. The mean normalised rate of flow of gas in the flame tube at the level of the mouth of the burner is 0.7 m/s. After cooling the reaction gases, the fumed silica powder is separated from the hydrochloric acid-containing gases using a filter and/or a cyclone. The fumed silica powder C is treated with water vapour and air in a deacidification unit.

The analytical data for powders 1 to 3 are given in the Tables.

**Table 1:**

| **Experimental conditions and the flame parameters for examples A to C** | | | | |
|---|---|---|---|---|
| **Example** | | **A** | **B** | **C** |
| SiCl₄ | kg/h | 400 | 400 | 500 |
| H₂ core | Nm³/h | 195 | 195 | 145 |
| Air | Nm³/h | 200 | 300 | 207 |
| O₂ content of air | Vol% | 30 | 29,5 | 35 |
| lambda | | 1.0 | 1.0 | 1.1 |
| gamma | | 1.8 | 1.8 | 1.0 |
| Mean speed of discharge at the mouth of the burner (normalised) | m/s | 47 | 47 | 49 |
| Mean speed of gas in the flame tube at the level of the mouth of the burner (normalised) | m/s | 10 | 9 | 0.7 |
| Throughput | kg/m³(*) | 0.31 | 0.26 | 0.42 |

| | | | | |
|---|---|---|---|---|
| (*) kg SiO₂/m³ of core gases | | | | |

**Table 2:**

| **Analytical data for silica powders A to C** | | | | |
|---|---|---|---|---|
| **Example** | | **A** | **B** | **C** |
| BET | m²/g | 45 | 44 | 44 |
| DBP | Nm³/h | 67 | 72 | 106 |
| Compacted bulk density | g/l | 117 | 105 | 112 |
| viscosity^{(#)} | mPas | 20 | 33 | 420 |
| pH | | 4.7 | 4.8 | 4.5 |
| Mean area(*) | nm² | 17063 | 15972 | 23217 |
| Mean circumf.(*) | nm | 742 | 658 | 1032 |
| Diameter max.(*) | nm | 191 | 183 | 292 |
| Diameter min.(*) | nm | 123 | 117 | 207 |

| | | | | |
|---|---|---|---|---|
| #: 30 wt.% dispersion at 5 rpm | | | | |
| * : Aggregate structure determined by image analysis. | | | | |

Powders according to the invention in examples A and B show lower values for all the parameters in Table 3 and thus have much lower structure than the powder C.

### Dental composite materials

### Example 1 (according to the invention)

50 parts of Diurethane Dimethacrylate (Plex 6661-0, Degussa) and 20 parts of fumed silica powder A were mixed with a Speed Mixer. After complete incorporation of the silica 30 parts of treated glass are added into the mixture. The resulting formulation shows a flowing and spreading behaviour with a viscosity of 384 Pas. The yield point was about 20 Pa. The rheological properties were determined with a rheometer based on cone/plate measuring system at a temperature of 23 °C.

**Examples 2 to 4** (according to the invention) were performed according to Example 1 but using the amount of fumed silica and treated glas given in Table 3.

### Example 5 (according to the invention)

50 parts of an acrylic resin mixture consisting of 80 parts Diurethane Dimethacrylate (Plex 6661-0, Degussa), 20 parts Triethyleneglycoldimethacrylate, 0,8 parts 2-Hydroxy-4-methoxybenzophenone, 0,12 parts Camphorquinone, 0,6 parts p-octyl dimethylamino-benzoate, and 20 parts of fumed silica powder A were mixed with a Speed Mixer. After complete incorporation of the silica 30 parts of treated glass are added into the mixture. The resulting formulation shows a flowing and spreading behaviour with a viscosity of 198 Pas. The yield point was about 703 Pa. The rheological properties were determined with a rheometer based on cone/plate measuring system at a temperature of 23 °C.

### Example 6 (according to the invention)

According to Example 5, using fumed silica powder B instead of A.

### Example 7 (comparison)

According to Example 5, using fumed silica powder C instead of A. The resulting formulation showed a plastic behaviour. The viscosity was 917 Pas and the yield point was about 1051 Pa.

**Table 3:**

| **Uncured dental composite materials** | | | | |
|---|---|---|---|---|
| | **Ex.1** | **Ex.2** | **Ex.3** | **Ex.4** |
| Fumed Silica A (parts) | 20 | 25 | 30 | 40 |
| Treated Glass (parts) | 30 | 25 | 20 | 10 |
| Viscosity [Pa x s] | 384 | 994 | 2136 | 2206 |
| Yield Point [Pa] | 20 | 25 | _* | _* |

| | | | | |
|---|---|---|---|---|
| * was not determined | | | | |

**Table 4:**

| **Uncured dental composite materials** | | | |
|---|---|---|---|
| | **Ex.5** | **Ex.6** | **Ex.7** |
| Fumed Silica | A | B | C |
| Fumed Silica (parts) | 20 | 20 | 20 |
| Treated Glass | 30 | 30 | 30 |
| Viscosity [Pa s] | 198 | 207 | 917 |
| Yield Point [Pa] | 703 | 723 | 1051 |

## Claims

1. Homogeneous dental composite material comprising fumed silica powder and a resin, the resin comprising a polymerizable monomer, **characterized in that** the fumed silica powder
- has a BET surface area 30 to 90 m²/g, a DBP number of 80 or less, an average aggregate area of less than 25,000 nm², and in which at least 70% of the aggregates have a diameter of less than 1300 nm and
- is present from at least 10 wt.%, based on the total dental composite material.

2. Homogeneous dental composite material according to claim 1, **characterized in that** it contains 15 to 50 wt.% of the fumed silica powder, based on the total composite material.

3. Homogeneous dental composite material according to claim 1, **characterized in that** it contains 20 to 35 wt.% of the fumed silica powder, based on the total composite material.

4. Homogeneous dental composite material according to claims 1 to 3, **characterized in that** it contains additional fillers.

5. Homogeneous dental composite composite material according to claims 1 to 4, **characterized in that** the polymerizable monomer is an acrylate or a mixture of acrylates.

6. Homogeneous dental composite material according to claim 1 to 5, **characterized in that** the resin consists of 60-90 parts of Diurethane Dimethacrylate, 10-40 parts Triethyleneglycoldimethacrylate, 0.4-1.0 parts 2-Hydroxy-4-methoxybenzophenone, 0.07-0.4 parts Camphorquinone, and 0.1-1.0 parts p-octyl dimethylamino-benzoate.

7. A method for preparing homogeneous dental composite material according to claim 1 to 6 comprising the steps:
- combining a resin comprising a polymerizable monomer and the fumed silica powder
- optionally adding additional fillers and
- optionally curing the mixture.
